(19) Europäisches Patentamt
European Patent Office
Office européen des brevets

(11) Veröffentlichungsnummer: **0 002 234**
**B1**

(12) # EUROPÄISCHE PATENTSCHRIFT

(21) Anmeldenummer: **78101445.1**

(22) Anmeldetag: **23.11.78**

(51) Int. Cl.³: **C 07 D 307/93,**
A 61 K 31/34

(54) **Neue Prostacyclinderivate, ihre Herstellung und Anwendung.**

(30) Priorität: **25.11.77 DE 2753244**

(43) Veröffentlichungstag der Anmeldung:
**13.06.79 Patentblatt 79/12**

(45) Bekanntmachung des Hinweises auf die Patenterteilung:
**11.02.81 Patentblatt 81/6**

(84) Benannte Vertragsstaaten:
**BE CH DE FR GB LU NL SE**

(56) Entgegenhaltungen:
**Journal of the American Chemical Society, 99, 1977, Seiten 2006—2008**

(73) Patentinhaber: **SCHERING AKTIENGESELLSCHAFT Berlin und Bergkamen
Müllerstrasse 170/178 Postfach 65 0311
D-1000 Berlin 65 (DE)**

(72) Erfinder: **Vorbrüggen, Helmut, Prof. Dr.
Wilkestrasse 7
D-1000 Berlin 27 (DE)**

(72) Erfinder: **Skuballa, Werner, Dr.
Olwenstrasse 25
D-1000 Berlin 28 (DE)**

(72) Erfinder: **Radüchel, Bernd, Dr.
Gollanczstrasse 132
D-1000 Berlin 28 (DE)**

(72) Erfinder: **Losert, Wolfgang, Prof. Dr.
Niedstrasse 12
D-1000 Berlin 41 (DE)**

(72) Erfinder: **Loge, Olaf, Dr.
Bekassinen Weg 37
D-1000 Berlin 27 (DE)**

(72) Erfinder: **Müller, Bernd, Dr.
Sonnenscheinstrasse 30
D-5100 Aachen (DE)**

(72) Erfinder: **Mannesmann, Gerda, Dr.
Hagenplatz 3e
D-1000 Berlin 33 (DE)**

EP 0 002 234 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Neue Prostacyclinderivate, ihre Herstellung und Anwendung

Die Erfindung betrifft neue Prostacyclin-Derivate, Verfahren zu ihrer Herstellung sowie ihre Verwendung als Arzneimittel.

Aus verschiedenen kürzlich erschienenen Publikationen (Nature [London] *263,* 663 (1976); Prostaglandins *14,* 210 (1977)) geht hervor, daß Prostaglandin-$I_2$ (PG $I_2$) die ADP-induzierte Blutplättchenaggregation hemmt. Außerdem wirkt PG-$I_2$ blutdrucksenkend aufgrund seiner dilatierenden Wirkung auf die glatte Muskulatur von Arterien.

PG $I_2$ besitzt jedoch nicht die für ein Arzneimittel notwendige Stabilität. So beträgt die Halbwertszeit von PG $I_2$ bei physiologischen pH-Werten und bei Raumtemperatur nur wenige Minuten.

Es wurde nun gefunden, daß die Einführung einer Nitrilgruppe an die Enolätherdoppelbindung zu einer Stabilisierung des Prostacyclins führt, wobei das pharmakologische Wirkungsspecktrum erhalten bleibt und die Wirkungsdauer deutlich verlängert wird.

Die erfindungsgemäßen Verbindungen wirken blutdrucksenkend. Außerdem sind diese Verbindungen zur Inhibierung der Thrombozytenaggregation geeignet.

Die Erfindung betrifft Prostanderivate der allgemeinen Formel I

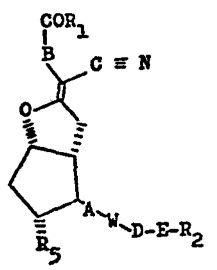

I

worin

$R_1$ den Rest $OR_3$, wobei $R_3$ Wasserstoff oder Alkyl mit 1—10 C-Atomen bedeuten kann, oder den Rest $NHR_4$ mit $R_4$ in der Bedeutung eines Alkanoyl- oder Alkansulfonylrestes mit je 1—10 C-Atomen darstellt,

B eine geradkettige Alkylengruppe mit 1—5 C-Atomen,

A eine $—CH_2—CH_2—$, cis-CH=CH— oder trans-CH=CH—Gruppe,

W eine

$$\underset{\substack{\wr \\ OH}}{\overset{\substack{H \\ \wr}}{—C—}} \text{ oder eine } \underset{\substack{\wr \\ OH}}{\overset{\substack{CH_3 \\ \wr}}{—C—}} \text{Gruppe, in denen}$$

die OH-Gruppe jeweils mit einem Alkansäurerest mit 1—4 C-Atomen verestert sein kann, wobei die freie oder veresterte OH-Gruppe $\alpha$- oder $\beta$-ständig sein kann,

D und E gemeinsam eine direkte Bindung oder

D eine geradkettige oder verzweigte Alkylengruppe mit 1—5 C-Atomen,

E eine direkte Bindung

$R_2$ eine gesättigte gerad- oder verzweigtkettige Alkylgruppe mit 1—6 C-Atomen, die durch Phenyl substituiert sein kann, oder eine Phenylgruppe,

$R_5$ eine Hydroxygruppe, die mit einem Alkansäurerest mit 1—4 C-Atomen verestert sein kann und, falls $R_3$ Wasserstoff darstellt, deren Salze mit physiologisch verträglichen Basen bedeuten.

Als Alkylgruppe $R_3$ sind gerade oder verzweigte Alkylgruppen mit 1—10 C-Atomen zu betrachten, wie beispielsweise Methyl, Äthyl, Propyl, Butyl, Isobutyl, tert.- Butyl, Pentyl, Neopentyl, Heptyl, Hexyl, Decyl.

Als bevorzugte Alkylgruppen $R_3$ sind solche mit 1—4 C-Atomen, wie z.B. Methyl, Äthyl, Propyl, Isobutyl, Butyl zu nennen.

Als Alkanoylrest $R_4$ kommen physiologisch verträgliche Säurereste in Frage, die sich von folgenden Carbonsäuren ableiten: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Trimethylessigsäure, Diäthylessigsäure, tert.-Butylessigsäure. Die Alkansulfonylreste $R_4$ leiten sich beispielsweise von Methansulfonsäure, Äthansulfonsäure, Isopropansulfonsäure, Butansulfonsäure ab.

Als Alkanoylreste von W und $R_5$ seien namentlich Acetyl, Propionyl und Butyryl genannt.

Als Alkylgruppe $R_2$ kommen gerad- und verzweigtkettige gesättigte Alkylreste mit 1—6 C-Atomen infrage, die durch Phenyl substituiert sein können. Beispielsweise genannt seien Methyl, Äthyl, Propyl, Butyl, Isobutyl, tert.-Butyl, Pentyl, Hexyl und Benzyl.

Als Alkylengruppe B kommen geradkettige gesättigte Alkylenreste mit 1—5 C-Atomen infrage. Namentlich seien genannt Methylen, Äthylen, Trimethylen, Tetramethylen, Pentamethylen.

Zur Salzbildung sind anorganische und organische Basen geeignet, wie sie dem Fachmann zur Bildung physiologisch verträglicher Salze bekannt sind. Beispielsweise seien genannt Alkalihydroxide wie Natrium- und Kaliumhydroxid, Erdalkalihydroxide wie Calciumhydroxid, Ammoniak, Amine wie Äthanolamin, Diäthanolamin, Triäthanolamin, N-Methylglucamin, Morpholin, Tris-(hydroxymethyl)-methylamin usw.

Die Erfindung betrifft ferner ein Verfahren zur Herstellung der erfindungsgemäßen Prostacyclinderivate der allgemeinen Formel I, dadurch gekennzeichnet, daß man in an sich bekannter Weise eine Verbindung der allgemeinen Formel II

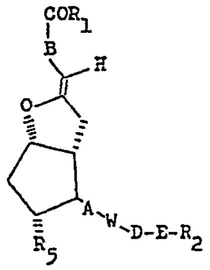

II

worin
$R_1$, $R_2$, $R_5$ A, B, W, D und E die oben angegebenen Bedeutungen haben, gegebenenfalls nach Schutz anwesender freier Hydroxygruppen mit einem Sulfonylisocyanat der allgemeinen Formel III

$$R_6SO_2NCO \qquad III$$

worin
$R_6$ einen Phenylring, der durch Alkyl mit 1—4 C-Atomen substituiert sein kann oder Halogen bedeutet und anschließend mit einem tertiären Amin oder tertiären Amid umsetzt.

Gegebenenfalls kann man anschließend in den erhaltenen Verfahrensprodukten in beliebiger Reihenfolge geschützte Hydroxygruppen freisetzen und/oder freie Hydroxygruppen verestern, eine veresterte Carboxylgruppe verseifen oder eine Carboxylgruppe verestern, eine Carboxylgruppe mit Verbindungen der allgemeinen Formel IV

$$O{=}C{=}N{-}R_4 \qquad IV,$$

worin
$R_4$ die oben angegebene Bedeutung hat, umsetzen oder eine Carboxylgruppe mit einer physiologisch verträglichen Base in ein Salz überführen.

Die Umsetzung der Enoläther II zu den Verbindungen der allgemeinen Formel I erfolgt nacheinander im gleichen Reaktionsgefäß mit Sulfonylisocyanten der Formel III und einem tertiären Amin in einem inerten Lösungsmittel, vorzugsweise Tetrahydrofuran, Dioxan, Diäthyläther, Toluol, bzw. tert. Amid, vorzugsweise ohne Lösungsmittel. Unter den Sulfonylisocyanten der allgemeinen Formel III seien genannt Benzolsulfonylisocyanat, p-Toluolsulfonylisocyanat sowie die Halogensulfonylisocyanate mit den Halogenen Fluor, Chlor und Brom. Besonders bevorzugt für die Reaktion mit Verbindungen der Formel II ist das Chlorsulfonylisocyanat.

Die Reaktion mit Sulfonylisocyanaten der Formel III erfolgt bei Temperaturen zwischen 30°C und —100°C, vorzugsweise zwischen —70°C und 0°C. Die Umsetzung mit einem tertiären Amin oder tertiären Amid erfolgt bei Temperaturen zwischen —100°C und 30°C, vorzugsweise zwischen —70°C und +30°C. Als tertiäre Amine kommen beispielsweise infrage: Triäthylamin, Trimethylamin, Diäthylisopropylamin, Dimethylisopropylamin, DBN, DBU usw. Als tertiäres Amid kommt vorzugsweise Dimethylformamid infrage.

Die Verseifung der Prostacyclinester wird nach den dem Fachmann bekannten Methoden durchgeführt, wie beispielsweise mit basischen Katalysatoren.

Die Einführung der Estergruppe $OR_3$ für $R_1$, bei welcher $R_3$ eine Alkylgruppe mit 1—10 C-Atomen darstellt, erfolgt nach den dem Fachmann bekannten Methoden. Die Carboxyverbindungen werden Beispielsweise mit Diazokohlenwasserstoffen in an sich bekannter Weise umgesetzt. Die Veresterung mit Diazokohlenwasserstoffen erfolgt zum Beispiel dadurch, daß man eine Lösung des Diazokohlenwasserstoffes in einem inerten Lösungsmittel, vorzugsweise in Diäthyläther mit der Carboxyverbindung in dem gleichen oder in einem anderen inerten Lösungsmittel, wie zum Beispiel Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden /Org. Reactions Bd. *8*, Seiten 389—394 (1954)/.

Die Prostacyclinderivate der allgemeinen Formel I mit $R_1$ in der Bedeutung einer Hydroxygruppe können mit geeigneten Mengen der entsprechenden anorganischen Basen unter Neutralisierung in Salze überführt werden. Beispielsweise erhält man beim Lösen des entsprechenden Prostacyclins in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Zur Herstellung eines Aminsalzes, die in üblicher Weise erfolgt, wird das Prostacyclin zum Beispiel in einem geeigneten Lösungsmittel, beispielsweise Äthanol, Aceton, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u.a., umsetzt.

Die Verseifung des Acylgruppe erfolgt beispielsweise mit Alkali- oder Erdalkalicarbonaten oder -hydroxiden in einem Alkohol oder der wäßrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei

−10°C bis 70°C, vorzugsweise bei 25°C.

Die Umsetzung der Verbindung der allgemeinen Formel I mit $R_3$ in der Bedeutung eines Wasserstoffatoms mit einem Isocyanat der allgemeinen Formel IV erfolgt gegebenenfalls unter Zusatz eines tertiären Amins, wie zum Beispiel Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Benzol, Toluol, Dimethylsulfoxid, bei Temperaturen ober- oder unterhalb Raumtemperatur, zum Beispiel zwischen −80°C bis 100°C, vorzugsweise bei 0°C bis 30°C, vorgenommen werden.

Enthält das Ausgangsprodukt OH-Gruppen, so werden diese Oh-Gruppen auch zur Reaktion gebracht. Werden letztlich Endprodukte gewünscht, die freie Hydroxylgruppen im Prostacyclin enthalten, geht man zweckmäßigerweise von Ausgangsprodukten aus, in denen diese durch vorzugsweise leicht abspaltbare Acylreste intermediär geschützt sind.

Die als Ausgangsmaterial dienenden Verbindungen der allgemeinen Formel II können beispielsweise hergestellt werden, indem man in an sich bekannter Weise ein bekanntes Prostaglandin F-Derivat der allgemeinen Formel V

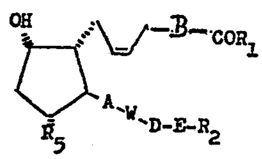

mit Jod in Gegenwart eines Alkalihydrogencarbonats oder Alkalicarbonats zu den Verbindungen der allgemeinen Formel VI

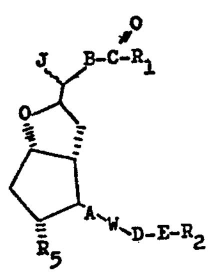

VI

umsetzt.

Anschließend kann man gegebenenfalls freie Hydroxygruppen durch Veresterung oder Silylierung schützen. Je nach der gewünschten Bedeutung von A oder $R_1$ in den Endprodukten der allgemeinen Formel I kann man gegebenenfalls Doppelbindungen in VI hydrieren oder gegebenenfalls eine Carboxygruppe verestern oder eine Carboxygruppe mit Verbindungen der allgemeinen Formel IV umsetzen.

Die Umsetzung der Verbindungen der allgemeinen Formel VI zu den Verbindungen der allgemeinen Formel II kann beispielsweise mit 1,5-Diazabicyclo [4.3.0]nonen-5 (DBN) oder 1,5-Diazabicyclo [5.4.0]undecen-5 (DBU) in einem inerten Lösungsmittel wie Benzol; Toluol, Tetrahydrofuran usw. oder mit Natriummethylat in Methanol erfolgen. Die Halogenwasserstoffabspaltung wird bei Temperaturen zwischen 0°C und 120°C, vorzugsweise bei 20—60°C durchgeführt.

Die neuen Prostacyclinderivate der allgemeinen Formel I sind wertvolle Pharmake, da sie bei ähnlichem Wirkungsspektrum gegenüber entsprechenden Prostaglandinen durch bessere Spezifität und vor allem durch eine wesentlich längere Wirkung auszeichnen. Im Vergleich zu PGE, PGA und PGI zeichnen sich die neuen Prostaglandine durch größere Stabilität aus, Die gute Gewebsspezifität der neuen Prostaglandine zeigt sich bei der Untersuchung an glattmuskulären Organen wie z.B. am Meerschweinchenileum oder an der isolierten Kaninchentrachea, wo eine wesentlich geringere Stimulation zu beobachten ist als bei Applikation natürlicher Prostaglandine.

Die neuen Prostaglandin-Analoga besitzen die für die Prostaglandine typischen pharmakologischen Eigenschaften, wie beispielsweise Senkung des Blutdrucks, Inhibierung der Thrombozytenaggregation, Hemmung der Magensäuresekretion u.a. Die Verabreichung der beanspruchten Verbindungen für die obengenannten Wirkungsweisen erfolgt in Dosisbereichen von 5 bis 1000 µg/kg/Tag, bevorzugt von 20 bis 250 µg/kg/Tag.

Bei intravenöser Injektion an wachen hypertonen Ratten in Dosen von 20 und 100 µg/kg Körpergewicht zeigen die erfindungsgemäßen Verbindungen eine stärkere blutdrucksenkende Wirking als $PGE_2$ und $PGA_2$, ohne wie $PGE_2$ in diesen Dosierungen Durchfälle oder wie $PGA_2$ kardiale Arrhythmien auszulösen.

Bei intravenöser Injektion an narkotisierten Kaninchen zeigen die erfindungsgemäßen Verbindungen im Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Während $PGI_2$ im Bronchospasmolyse-Test bereits mit 50 µg/ml gegenüber der Kontrollgruppe keine signifikant bessere Wirksamkeit mehr zeigt, wirkt 5 - Cyano - 16 - methyl - prostacyclin noch mit 2 µg/ml. Im Blutdrucktest besitzt dieselbe Verbindung bei einer Dosis von 50 µg/kg an der Ratte einen mit $PGI_2$ vergleichbaren maximalen blutdrucksenkenden Effekt, verlängert dafür aber die blutdrucksenkende Wirkung um das 2—5 fache. Außerdem verringern sich die Nebenwirkungen (z.B. Rhythmus-störungen, Apathie) bei den beanspruchten Verbindungen deutlich.

Für die parenterale Verabreichung werden sterile, injizierbare, wäßrige oder ölige Lösungen benutzt.

Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen, zum Beispiel zur Herstellung von Blutdrucksenkern dienen.

Beispiel 1

5 - Cyano - prostacyclin - methylester - 11,15 - diacetat

Zu einer Lösung von 320 mg Prostacyclin-methylester-11,15-diacetat in 4,2 ml abs. Äther tropft man bei −70°C 3,55 ml einer Chlorsulfonylisocyanatlösung (Herstellung: man löst 2,3 ml Chlorsulfonylisocyanat in 50 ml abs. Äther), erwärmt langsam auf 0°C und tropft 3,55 ml einer Lösung von Triäthylamin (Herstellung: 100,2 mg Triäthylamin in 5 ml Methylenchlorid) zu. Man rührt 1 Stunde bei 0°C, 15 min bei 20°C, gießt auf eine Mischung aus Natriumbicarbonatlösung und Eiswasser, extrahiert dreimal mit Äther, schüttelt den org. Extrakt zweimal mit Sole, trocknet über Magnesiumsulfat und dampft im Vakuum ein. Nach Reinigung durch präparative Dünnschichtchromatographie (PDC) (Kieselgel, Äther) erhält man 60 mg der Titelverbindung als farbloses Öl.

IR ($CHCl_3$): 2959, 2930, 2860, 2203, 1730, 1650, 1372, 1245, 970/cm.

Das Ausgangsmaterial für die Titelverbindung wurde wie folgt hergestellt:

1a) 5,6 - Dihydro - 5 - jod - prostacyclin - methylester

Zu einer Mischung von 2,16 g Prostaglandin - $F_{2\alpha}$ - methylester, 5.40 g Natriumbikarbonat, 50 ml Äther und 90 ml Wasser tropft man unter Rühren bei 0°C innerhalb von 3 Stunden 65,2 ml einer 2,5 %igen ätherischen Jod- Lösung. Nach 22 Stunden bei 0°C verdünnt man mit Äther, schüttelt mit verd. Natriumthiosulfatlösung, wäscht mit Wasser neutral, trocknet über Magnesiumsulfat und dampft im Vakuum ein. Nach Filtration über Kieselgel mit Äther/Essigester (1 + 1) erhält man 2,81 g der Titelverbindung als farbloses Öl.

IR ($CHCl_3$): 3600, 3400, 2932 1730, 795/cm

1b) 5,6 - Dihydro - 5 - jod - prostacyclin - methylester - 11,15 - diacetat

Man löst 400 mg des nach Beispiel 1a hergestellten Diels in 0,8 ml Essigsäureanhydrid und 3 ml Pyridin und läßt 18 Stunden bei Raumtemperatur stehen. Nach Eindampfen im Vakuum erhält man 467 mg des DC-einheitlichen Diacetates als farbloses Öl.

IR ($CHCl_3$): 2958, 2948, 2860, 1732, 1372, 1245, 976/cm

1c) Prostacyclin - methylester - 11,15 - diacetat

Zu einer Lösung von 200 mg des nach 1b hergestellten Diacetates in 2 ml Benzol fügt man 1 ml 1,5-Diazabicyclo[4.3.0]non - 5 - en (DBN) und rührt 20 Stunden bei 40°C unter Argon. Man verdünnt mit Äther, schüttelt dreimal mit Eiswasser, trocknet über Natriumsulfat und engt im Vakuum bei 20°C ein. Dabei erhielt man die ölige Titelverbinding, die ohne weitere Reinigung verwendet wird.

Beispiel 2

5 - Cyano - prostacyclin - methylester

250 mg der nach Beispiel 1 hergestellten Verbindung, 150 mg Kaliumcarbonat und 10 ml Methanol rührt man 3,5 Stunden bei Raumtemperatur unter Argon. Anschließend verdünnt man mit Äther, wäscht mit Wasser neutral, trocknet über $MgSO_4$ und dampft im Vakuum bei 25°C ein.

IR ($CHCl_3$): 3600, 3430, 2937, 2860, 2212, 1730, 1650, 972/cm

Beispiel 3

5 - Cyan - prostacyclin

Zu einer Lösung von 25 mg der nach Beispiel 2 hergestellten Verbindung in 1,5 ml Methanol fügt man 0,25 ml 1 n Natronlauge und rührt 4 Stunden bei 25°C unter Argon. Anschließend engt man im Vakuum ein, nimmt in 3 ml Sole auf und stellt die Lösung mit 0,5 %iger Zitronensäure auf pH 7 ein. Man extrahiert viermal mit Methylenchlorid, schüttelt den organischen Extrakt einmal mit Sole, trocknet über Magnesiumsulfat und dampft im Vakuum ein. Dabei erhält man 22 mg der DC-einheitlichen Titelverbindung als Öl, das beim Aufbewahren bei −20°C kristallisiert.

IR ($CHCl_3$): 3600, 2930, 2862, 2211, 1710, 1650, 973/cm

Beispiel 4

5 - Cyano - 15 - methyl - prostacyclin - methylester - 11,15 - diacetat

Zu einer Lösung von 644 mg 15 - Methyl - prostacyclin - methylester - 11,15 - diacetat in 9 ml abs. Äther tropft man bei −70°C 7,2 ml einer Chlorsulfonylisocyanatlösung (Herstellung: man löst 2,3 ml Chlorsulfonylisocyanat in 50 ml abs. Äther), erwärmt langsam auf 0°C und tropft 7,2 ml einer Lösung von Triäthylamin (Herstellung: 200 mg Triäthylamin in 10 ml Methylenchlorid) zu. Man rührt 1 Stunde bei 0°C, 15 min bei 20°C, gießt auf eiskalte Natriumbicarbonatlösung, extrahiert dreimal mit Äther, schüttelt den organischen Extrakt zweimal mit Sole, trocknet über Magnesiumsulfat und dampft im Vakuum ein. Nach Reinigung durch PDC (Äther) erhält man 135 mg der Titelverbindung als farbloses Öl.

IR ($CHCl_3$): 2960, 2930, 2203, 1730, 1650, 1245, 972/cm

Das Ausgangsmaterial für die Titelverbindung wurde wie folgt hergestellt:

4a) 5,6 - Dihydro - 5 - jod - 15 - methyl - prostacyclin - methylester

Zu einer Mischung von 1,1 g 15 - Methyl - Prostaglandin - $F_{2\alpha}$ - methylester, 2,70 g

Natriumbicarbonat, 30 ml Äther und 50 ml Wasser tropft man unter Rühren bei 0°C innerhalb von 3 Stunden 33 ml einer 2,5 %igen ätherischen Jod- lösung. Nach 23 Stunden bei 0°C verdünnt man mit Äther, schüttelt mit Natriumthiosulfatlösung, wäscht mit Wasser neutral, trocknet über Magnesiumsulfat und dampft im Vakuum ein. Nach Filtration über Kieselgel mit Äther/Essigester (1 + 1) erhält man 1,35 g der Titelverbindung als Öl.
IR ($CHCl_3$): 3600, 3400, 1730, 976/cm

4b) 5,6 - Dihydro - 5 - jod - 15 - methyl - prostacyclin - methylester - 11,15 - diacetat

Man löst 1,30 g der nach Beispiel 4a hergestellten Verbindung in 12 ml Pyridin und 3 ml Essigsäureanhydrid, fügt 100 mg 4 - Dimethylaminopyridin hinzu und läßt 16 Stunden bei 25°C stehen. Anschließend dampft man im Vakuum ein und filtriert den Rückstand über Kieselgel mit Pentan/Äther (8 + 2) und erhält 1,41 g der Titelverbindung als farbloses Öl.
IR ($CHCl_3$): 2960, 2860, 1733, 1245, 976/cm

4c) 15 - Methyl - prostacyclin - methylester - 11,15 - diacetat

Eine Mischung aus 600 mg des nach 4b hergestellten Diacetates. 6 ml Benzol und 3 ml DBN rührt man 20 Stunden bei 45°C unter Argon. Anschließend verdünnt man mit Äther, schüttelt dreimal mit Eiswasser, trocknet über Natriumsulfat und engt im Vakuum bei 20°C ein. Man erhält die ölige Titelverbindung, die ohne weitere Reinigung verwendet wird.

Beispiel 5

5 - Cyano - 15 - methyl - prostacyclin

Zu einer Lösung von 100 mg der nach Beispiel 4 hergestellten Verbindung in 6 ml Methanol, fügt man 1 ml 2 n Natronlauge und rührt 5 Stunden bei 25°C unter Argon. Anschließend engt man im Vakuum ein, nimmt in 5 ml Sole auf, stellt mit 0,5 %iger Zitronensäure auf pH 7 ein und extrahiert viermal mit Methylenchlorid, schüttelt den organ. Extrakt einmal mit Sole, trocknet über Magnesiumsulfat und dampft im Vakuum ein. Das Rohprodukt wird durch Filtration über Kieselgel mit Methylenchlorid/Isopropanol (85 + 15) gereinigt. Man erhält 56 mg der Titelverbindung als farbloses Öl.
IR ($CHCl_3$): 3600, 3300, 2930, 2862, 2210, 1712, 1650, 974/cm

Beispiel 6

5 - Cyano - 16,16 - dimethyl - prostacyclin - methylester - 11,15 - diacetat

Zu einer Lösung von 320 mg 16, 16 - Dimethyl - prostacyclin - methylester - 11,15 - diacetat in 4,5 ml Äther tropft man bei −70°C 3,5 ml einer Chlorsulfonylisocyanatlösung (Herstellung: Beispiel 1), erwärmt langsam auf 0°C und tropft 3,5 ml einer Lösung von Triäthylamin (Herstellung: Beispiel 1) zu. Man rührt eine Stunde bei 0°C, 15 min bei 20°C, gießt auf eiskalte Natriumbicarbonatlösung, extrahiert dreimal mit Äther, schüttelt den organischen Extrakt zweimal mit Sole, trocknet über Magnesiumsulfat und dampft im Vakuum ein. Nach Reinigung durch PDC (Äther) erhält man 72 mg der Titelverbindung als farbloses Öl.
IR ($CHCl_3$): 2960, 2930, 2204, 1732, 1650, 1245, 972/cm

Das Ausgangsmaterial für die Titelverbindung wurde wie folgt hergestellt:

6a) 5,6 - Dihydro - 16,16 - dimethyl - 5 - jod - prostacyclin - methylester

In Analogie zu Beispiel 1a erhält man aus 1,2 g 16,16 - Dimethyl - Prostaglandin - $F_{2\alpha}$ - methylester 1,5 g der Titelverbindung als Öl.
IR ($CHCl_3$): 3600, 3400, 1730, 975/cm

6b) 5,6 - Dihydro - 16,16 - dimethyl - 5 - jod - prostacyclin - methylester - 11,15 - diacetat

Eine Lösung aus 820 mg des nach Beispiel 6a hergestellten Diols in 6 ml Pyridin und 1,5 ml Essigsäureanhydrid läßt man 18 Stunden bei Raumtemperatur stehen, dampft im Vakuum ein und filtriert den Rückstand mit Pentan/Äther (1 + 1) über Kieselgel. Man erhält 890 mg der Titelverbindung als farbloses Öl.
IR ($CHCl_3$): 2960, 2948, 1732, 1245, 975/cm

6c) 16,16 -Dimethyl - prostacyclin - methylester - 11,15 - diacetat

In Analogie zu Beispiel 1c erhält man aus 800 mg des nach Beispiel 6b hergestellten Diacetates und 4 ml DBN die Titelverbindung als Öl.

Beispiel 7

5 - Cyano - 16,16 - dimethyl - prostacyclin

Zu einer Lösung von 400 mg der nach Beispiel 6 hergestellten Verbindung in 25 ml Methanol fügt man 4 ml 2 n Natronlauge und rührt 6 Stunden bei 25°C unter Argon. Anschließend engt man im Vakuum ein, nimmt in 15 ml Sole auf, stellt mit 1 %iger Zitronensäure auf pH 7 ein und extrahiert viermal mit Methylenchlorid. Man schüttelt den organischen Extrakt einmal mit Sole, trocknet über Magnesiumsulfat und dampft im Vakuum ein. Das Rohprodukt wird durch Filtration über Kieselgel (Methylenchlorid/ Isopropanol 85 + 15) gereinigt. Man erhält 240 mg der Titelverbindung als farbloses Öl.
IR ($CHCl_3$): 3600, 3300, 2930, 2860, 2210, 1710, 1650, 975/cm

Beispiel 8

5 - Cyano - 16 - methyl - prostacyclin - methylester - 11,15 - diacetat

Zu einer Lösung von 1,30 g 16 - Methyl - prostacyclin - methylester - 11,15 - diacetat in 18 ml abs. Äther tropft man bei −70°C 14,4 ml einer Chlorsulfonylisocyanatlösung (Herstellung: man löst 2,3 ml Chlorsulfonylisocyanat in 50 ml abs. Äther), erwärmt langsam auf 0°C innerhalb von 30 min und tropft 14,4

einer Lösung von Triäthylamin (Herstellung: 400 mg Triäthylamin in 20 ml Methylenchlorid) zu. Man rührt eine Stunde bei 0°C, 15 min bei 20°C, gießt auf eiskalte Natriumbicarbonatlösung, extrahiert dreimal mit Äther, schüttelt den organischen Extrakt zweimal mit Sole, trocknet über Magnesiumsulfat und dampft im Vakuum ein. Nach Reinigung durch PDC (Äther) erhält man 290 mg der Titelverbindung als farbloses Öl.
IR ($CHCl_3$): 2960, 2932, 2203, 1730, 1650, 1250, 972/cm

Das Ausgangsmaterial für die Titelverbindung wurde wie folgt hergestellt:

8a) 5,6 - Dihydro - 5 - jod - 16 - methyl - prostacyclin - methylester

In Analogie zu Beispiel 1a erhält man aus 4,30 g 16 - Methyl - prostaglandin - $F_{2\alpha}$ - methylester 5,60 g der Titelverbindung als farbloses Öl.
IR ($CHCl_3$): 3600, 3400, 2930, 1732, 975/cm

8b) 5,6 - Dihydro - 5 - jod - 16 - methyl - prostacyclin - methylester - 11,15 - diacetat

Man löst 5,51 g der nach Beispiel 8a hergestellten Verbindung in 30 ml Pyridin und 8 ml Essigsäureanhydrid und läßt 18 Stunden bei 25°C stehen. Nach Eindampfen im Vakuum und Filtration über Kieselgel (Pentan/Äther 7 + 3) erhält man 6 g der Titelverbindung als farbloses Öl.
IR ($CHCl_3$): 2960, 2950, 2860, 1730, 1245, 975/cm

8c) 16 - Methyl - prostacyclin - methylester - 11,15 - diacetat

Eine Mischung aus 3 g der nach Beispiel 8b hergestellten Verbindung, 30 ml Benzol und 15 ml DBN rührt man 20 Stunden bei 45°C unter Argon, verdünnt mit Äther, schüttelt viermal mit Eiswasser, trocknet über Natriumsulfat und engt im Vakuum bei 20°C ein. Man erhält die ölige Titelverbindung, die ohne weitere Reinigung verwendet wird.

Beispiel 9

5 - Cyano - 16 - methyl - prostacyclin

Zu einer Lösung von 1 g der nach Beispiel 8 hergestellten Verbindung in 50 ml Methanol fügt man 10 ml 2 n Natronlauge und rührt 7 Stunden bei 25°C unter Argon. Anschließend engt man im Vakuum ein, nimmt in 50 ml. Sole auf, stellt mit 1 %iger Zitronensäure auf pH 7 ein und extrahiert viermal mit Methylenchlorid. Man schüttelt den organischen Extrakt einmal mit Sole, trocknet über Magnesiumsulfat und dampft im Vakuum ein. Nach Filtration des Rohproduktes über Kieselgel erhält man mit Methylenchlorid/Isopropanol (85 + 15) 590 mg der Titelverbindung als farbloses Öl.
IR ($CHCl_3$): 3600, 3300, 2933, 2860, 2210, 1710, 1650, 976/cm

Beispiel 10

5 - Cyano - 16 - phenyl - 17,18,19,20 - tetranor - prostacyclin - methylester - 11,15 - diacetat

Zu einer Lösung von 300 mg 16 - Phenyl - 17,18,19,20 - tetranor - prostacyclin - methylester - 11,15 - diacetat in 4 ml Äther tropft man bei −70°C 3,4 ml einer Chlorsulfonylisocyanatlösung (Herstellung siehe Beispiel 1), erwärmt innerhalb von 30 min auf 0°C und tropft 3,4 ml einer Triäthylaminlösung in Methylenchlorid (Herstellung siehe Beispiel 1) zu. Man rührt eine Stunde bei 0°C, 15 min bei 20°C, gießt auf eiskalte Natriumbicarbonatlösung extrahiert mit Äther, wäscht den Extrakt mit Sole neutral, trocknet über Magnesiumsulfat und dampft im Vakuum ein. Nach Reinigung durch PDC (Äther) erhält man 80 mg der Titelverbindung als Öl.
IR ($CHCl_3$): 2960, 2205, 1733, 1651, 1602, 1245, 794/cm

Das Ausgangsmaterial für die obige Titelverbindung wurde wie folgt hergestellt:

10a) 5,6 - Dihydro - 5 - jod - 16 - phenyl - 17,18,19,20 - tetranor - prostacyclin - methylester

In Analogie zu Beispiel 1a erhält man aus 1,05 g 16-Phenyl - 17,18,19,20 - tetranor - Prostaglandin - $F_{2\alpha}$ - methylester 1,38 g der Titelverbindung als farbloses Öl.
IR ($CHCl_3$): 3600, 3410, 1732, 1602, 975/cm

10b) 5,6 - Dihydro - 5 - jod - 16 - phenyl - 17,18,19,20 - tetranor - prostacyclin - methylester - 11,15 - diacetat

Eine Lösung von 1,20 g des nach Beispiel 10a hergestellten Diols in 9 ml Pyridin und 2,4 ml Essigsäureanhydrid läßt man 18 Stunden bei Raumtemperatur stehen. Anschließend dampft man im Vakuum ein und filtriert den Rückstand mit Pentan/Äther (1 + 1) über Kieselgel. Dabei erhält man 1,31 g der Titelverbindung als farbloses Öl.
IR ($CHCl_3$): 2958, 2950, 1732, 1602, 1245, 976/cm

10c) 16 - Phenyl - 17,18,19,20 - tetranor - prostacyclin - methylester - 11,15 - diacetat

In analogie zu Beispiel 1c erhält man aus 1,25 g des nach Beispiel 10b hergestellten Diacetates und 6 ml DBN die ölige Titelverbindung, die als Rohprodukt weiter verwendet wird.

Beispiel 11

5 - Cyano - 16 - phenyl - 17, 18, 19, 20 - tetranor - prostacyclin

Zu einer Lösung von 610 mg der nach Beispiel 10 hergestellten Verbindung in 28 ml Methanol fügt man 6 ml 2 n Natronlauge und rührt 6 Stunden bei 25°C unter Argon. Anschließend engt man im Vakuum ein, nimmt in 15 ml Sole

auf, stellt mit 1 %iger Zitronensäurelösung auf pH 7 ein und extrahiert viermal mit Methylenchlorid. Man schüttelt den organischen Extrakt einmal mit Sole, trocknet über Magnesiumsulfat und dampft im Vakuum ein. Nach Reinigung des Rohproduktes durch Filtration über Keiselgel (Methylenchlorid/Isopropanol 9 + 1) erhält man 390 mg der Titelverbindung als farbloses Öl.

IR ($CHCl_3$): 3600, 3310, 2930, 2860, 2212, 1712, 1650, 1602, 796/cm

Beispiel 12

5 - Cyano - 13,14 - dihydro - 16 - methyl - prostacyclin - methylester - 11,15 - diacetat

Zu einer Lösung von 1,15 g 13, 14 - Dihydro - 16 - methyl - prostacyclin - methylester - 11,15 - diacetat in 15 ml abs. Äther tropft man bei −70°C 13 ml einer Chlorsulfonylisocyanatlösung (Herstellung siehe Beispiel 1), erwärmt innerhalb von 30 min auf 0°C, tropft 13 ml einer Lösung von Triäthylamin in Methylenchlorid (siehe Beispiel 1) zu, rührt 1 Stunde bei 0°C, gießt auf eiskalte Natriumbicarbonatlösung und extrahiert mit Äther. Der Extrakt wird mit Sole, gewaschen, über Magnesiumsulfat getrocknet und im Vakuum bei 25°C eingedampft. Durch PDC (Äther) erhält man 300 mg der Titelverbindung als farbloses Öl.

IR ($CHCl_3$): 2960, 2932, 2203, 1730, 1650, 1250/cm

12a) 5,6 - Dihydro - 13,14 - dihydro - 5 - jod - 16 - methyl - prostacyclin - methylester

In Analogie zu Beispiel 1a erhält man aus 2,1 g 13, 14 - Dihydro - 16 - methyl - Prostaglandin - $F_{2\alpha}$ - methylester 2,6 g der Titelverbindung als farbloses Öl.

IR ($CHCl_3$): 3600 3400, 2930, 1730/cm

12b) 5,6 - Dihydro - 13,14 - dihydro - 5 - jod - 16 - methyl - prostacyclin - methylester - 11,15 - diacetat

In Analogie zu Beispiel 1b erhält man aus 2,50 g des nach Beispiel 12a hergestellten Diols nach Chromatographie 2,70 der Titelverbindung als Öl.

IR ($CHCl_3$): 2958, 2950, 2855, 1732, 1245/cm

12c) 13, 14 - Dihydro - 16 - methyl - prostacyclin - methylester - 11,15 - diacetat

In Analogie zu Beispiel 1c erhält man aus 2 g der nach Beispiel 12b hergestellten Verbindung und 10 ml DBN die Titelverbindung, die als Rohprodukt weiter verwendet wird.

Beispiel 13

5 - Cyano - 13,14 - dihydro - 16 - methyl - prostacyclin

In Analogie zu Beispiel 5 erhält man aus 250 mg der nach Beispiel 12 hergestellten Verbindung und 2,5 ml 2 n Natronlauge in 5 ml Methanol 145 mg der Titelverbindung als farbloses Öl.

IR ($CHCl_3$): 3600, 3300, 2930, 2862, 2212, 1710

Beispiel 14

5 - Cyano - N - methansulfonyl - prostacyclin - carboxamid

377 mg 5 - Cyano - prostacyclin (Herstellung siehe Beispiel 3), 3 ml Pyridin und 1 ml Essigsäureanhydrid läßt man 16 Stunden bei Raumtemperatur stehen und dampft im Vakuum ein. Den Rückstand löst man in 10 ml abs. Acetonitril, fügt 120 mg Triäthylamin und versetzt mit einer Lösung aus 150 mg Methylsulfonylisocyanat in 8 ml Acetonitril. Man rührt 4 Stunden bei 20°C, engt im Vakuum ein, versetzt mit 10 ml Wasser, stellt mit 1 %iger Zitronensäurelösung auf pH 7 ein und extrahiert mit Äther. Man schüttelt den organischen Extrakt mit Sole, trocknet über Magnesiumsulfat und dampft im Vakuum ein. Nach Reinigung durch PDC (Äther) erhält man 340 mg des Methansulfonylcarboxamids. Zur Abspaltung der Acetatschutzgruppen löst man in 10 ml Methanol, versetzt mit 240 mg Kaliumcarbonat und rührt 3 Stunden bei 20°C unter Argon. Anschließend verdünnt man mit Sole, stellt die Mischung mit 1 % iger Zitronensäurelösung auf pH 7 ein, extrahiert mit Methylenchlorid, schüttelt den Extrakt mit Sole, trocknet über Magnesiumsulfat und dampft im Vakuum ein.

Nach Filtration über Kieselgel mit Methylenchlorid/Isopropanol (9 + 1) erhält man 203 mg der Titelverbindung als farbloses Öl.

IR ($CHCl_3$): 3400, 2935, 2865, 2211, 1720, 1650, 1340, 975/cm

Beispiel 15

5 - Cyano - 16 - methyl - N - methansulfonyl - prostacyclin - carboxamid

In Analogie zu Beispiel 14 erhält man aus 250 mg 5 - Cyano - 16 - methyl - prostacyclin 173 mg der Titelverbindung als Öl.

IR ($CHCl_3$): 3400, 2940, 2865, 2210, 1718, 1650, 972/cm

Beispiel 16

5 - Cyano - N - acetyl - prostacyclin - carboxamid

190 mg 5 - Cyano - prostacyclin (siehe Beispiel 3), 1,5 ml Pyridin und 0,5 ml Essigsäureanhydrid läßt man 16 Stunden bei Raumtemperatur stehen und dampft dann im Vakuum ein. Den Rückstand löst man in 6 ml Acetonitril, fügt eine Lösung aus 75 mg Triäthylamin in 6 ml Acetonitril und versetzt bei 0°C mit einer Lösung aus 55 mg Acetylisocyanat in 6 ml Acetonitril. Man rührt 2 Stunden bei 20°C, engt im Vakuum ein, versetzt mit 10 ml Wasser, stellt mit 1%-iger. Zitronensäurelösung auf pH 7 ein und extrahiert mit Äther. Man schüttelt den Extrakt mit Sole, trocknet über Magnesiumsulfat und dampft im Vakuum ein. Nach Reinigung durch PDC (Äther/Pentan 7 + 3) erhält man 160 mg des Acetylcarboxyamids, Zur Abspaltung der Acetatschutzgruppen löst man in 5 ml Methanol, versetzt mit 105 mg Kaliumcarbonat und rührt 3 Stunden bei 20°C unter Argon. Anschließend verdünnt man mit Sole, stellt die Mischung mit 1 %iger Zitronensäurelösung auf pH 7 ein, extrahiert mit Methylenchlorid, schüttelt den Extrakt mit Sole, trocknet über Magnesiumsulfat und dampft im Vakuum ein. Nach Filtration über Kieselgel (Methylenchlorid/Isopropanol 9 + 1) erhält man 105 mg der Titelverbindung als Öl.
IR ($CHCl_3$): 3600, 3400, 2960, 2210, 1733, 1705, 1650, 973/cm

Beispiel 17

5 - Cyano - 15 - methyl - N - acetyl - prostacyclin - carboxamid

In Analogie zu Beispiel 16 erhält man aus 225 mg 5 - Cyano - 15 - methyl - prostacyclin (Beispiel 5) 152 mg der Titelverbindung als Öl.
IR ($CHCl_3$): 3600, 3400, 2955, 2212, 1733, 1706, 1650, 973/cm

Beispiel 18

5 - Cyano - 16,16 - dimethyl - N - acetyl - prostacyclin - carboxamid

In Analogie zu Beispiel 16 erhält man aus 170 mg 5 - Cyano - 16,16 - dimethyl - prostacyclin (Beispiel 7) 95 mg der Titelverbindung als Öl.
IR ($CHCl_3$): 3600, 3410, 2960, 2210, 1732, 1705, 1650, 976/cm

Beispiel 19

5 - Cyano - 16 - methyl - N - acetyl - prostacyclin - carboxamid

In Analogie zu Beispiel 16 erhält man aus 152 mg 5 - Cyano - 16 - methyl - prostacyclin (Beispiel 9) 102 mg der Titelverbindung als Öl.
IR ($CHCl_3$): 3600, 3410, 2955, 2210, 1735, 1708, 1650, 974/cm

Beispiel 20

5 - Cyano - N - acetyl - 16 - phenyl - 17,18,19,20 - tetranor - prostacyclin - carboxamid

In Analogie zu Beispiel 16 erhält man aus 165 mg 5 - Cyano - 16 - phenyl - 17,18,19,20 - tetranor - prostacyclin 100 mg der Titelverbindung als farbloses Öl.
IR ($CHCl_3$): 3600, 3400, 2945, 2212, 1734, 1708, 1650, 1602, 975/cm

Beispiel 21

5 - Cyano - 13,14 - dihydro - 16 - methyl - N - acetyl - prostacyclin - carboxamid

In Analogie zu Beispiel 16 erhält man aus 95 mg 5 - Cyano - 13,14 - dihydro - 16 - methyl - prostacyclin 57 mg der Titelverbindung als Öl.
IR ($CHCl_3$): 3600, 3400, 2955, 2210, 1734, 1706, 1650/cm

Beispiel 22

Tris - (hydroxymethyl) - aminomethansalz von 5 - Cyano - prostacyclin

Zu einer Lösung von 190 mg 5 - Cyano - prostacyclin in 7 ml Acetonitril gibt man bei 80°C unter Rühren eine Lösung von 60 mg Tris - (hydroxymethyl) - aminomethan in 0,2 ml Wasser und rührt 16 Stunden bei Raumtemperatur. Man erhält nach Abtrennen des Lösungsmittels 185 mg der Titelverbindung.

**Patentansprüche**

1. Prostacyclinderivate der allgemeinen Formel I

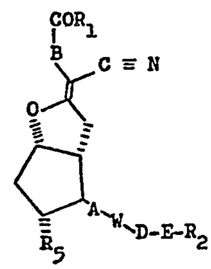

I

worin

$R_1$ den Rest $OR_3$, wobei $R_3$ Wasserstoff oder Alkyl mit 1—10 C-Atomen bedeuten kann, oder den Rest $NHR_4$ mit $R_4$ in der Bedeutung eines Alkanoyl- oder Alkansulfonylrestes mit je 1—10 C-Atomen darstellt,

B eine geradkettige Alkylengruppe mit 1—5 C-Atomen,

A eine $—CH_2—CH_2—$, cis-CH=CH— oder trans-CH=CH—Gruppe,

W eine

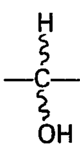 oder eine 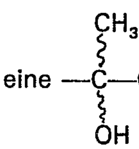 Gruppe, in denen

die OH-Gruppe jeweils mit einem Alkansäurerest mit 1—4 C-Atomen verestert sein kann, wobei die freie oder veresterte OH-Gruppe α- oder β-ständig sein kann,

D und E gemeinsam eine direkte Bindung oder

D eine geradkettige oder verzweigte Alkylengruppe mit 1—5 C-Atomen,

E eine direkte Bindung,

$R_2$ eine gesättigte gerad- oder verzweigtkettige Alkylgruppe mit 1—6 C-Atomen, die durch Phenyl substituiert sein kann, oder eine Phenylgruppe,

$R_5$ eine Hydroxygruppe, die mit einem Alkansäurerest mit 1—4 C-Atomen verestert sein kann und, falls $R_3$ Wasserstoff darstellt, deren Salze mit physiologisch verträglichen Basen bedeuten.

2. 5-Cyano-prostacyclin

3. 5-Cyano-16-methyl-prostacyclin

4. Verfahren zur Herstellung von Prostacyclinderivaten der allgemeinen Formel I, dadurch gekennzeichnet, daß man in an sich bekannter Weise eine Verbindung der allgemeinen Formel II

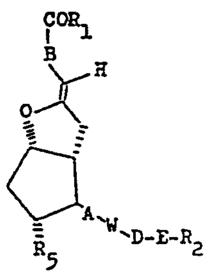

II

worin $R_1$, $R_2$, $R_5$ A, B, W, D und E die oben angegebenen Bedeutungen haben, gegebenenfalls nach Schutz anwesender freier Hydroxygruppen mit einem Sulfonylisocyanat der allgemeinen Formel III

$$R_6SO_2NCO \qquad III$$

worin $R_6$ einen Phenylring, der durch Alkyl mit 1—4 C-Atomen substituiert sein kann oder Halogen bedeutet und anschließend mit einem tertiären Amin oder tertiären Amid umsetzt.

5. Arzneimittel, bestehend aus einer oder mehreren Verbindungen gemäß Anspruch 1 und üblichen Hilfs- und Trägerstoffen.

**Claims**

1. Prostacyclin derivatives of the general formula I

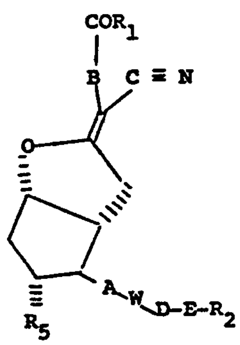

I

in which

$R_1$ represents the radical $OR_3$, wherein $R_3$ may represent hydrogen or alkyl having 1 to 10 carbon atoms, or the radical $NHR_4$ wherein $R_4$ represents an alkanoyl or alkanesulphonyl radical each having 1 to 10 carbon atoms,

B represents a straight-chain alkylene group having 1 to 5 carbon atoms,

A represents a $—CH_2—CH_2—$, *cis*-CH=CH— or *trans*-CH=CH— group,

W represents a

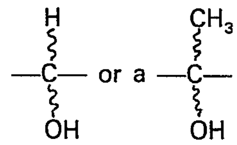

or a

group in which the OH group may in each case be esterified by an alkanoic acid radical having 1 to 4 carbon atoms, it being possible for the free or esterified OH group to be in the $\alpha$- or $\beta$-position,

D and E together represent a direct bond or

D represents a straight-chain or branched alkylene group having 1 to 5 carbon atoms and

E represents a direct bond,

$R_2$ represents a saturated straight-chain or branched-chain alkyl group having 1 to 6 carbon atoms that may be substituted by phenyl, or represents a phenyl group,

$R_5$ represents a hydroxy group that may be esterified by an alkanoic acid radical having 1 to 4 carbon atoms,

and, if $R_3$ represents hydrogen, the salts thereof with physiological tolerable bases.

2. 5-Cyanoprostacyclin.

3. 5-Cyano-16-methylprostacyclin.

4. Process for the manufacture of prostacyclin derivatives of the general formula I, characterised in that, in a manner known *per se*, a compound of the general formula II

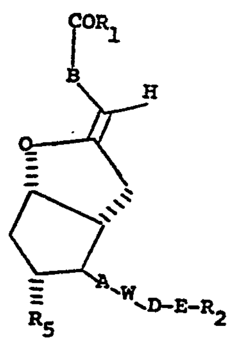

II

in which $R_1$, $R_2$, $R_5$, A, B, W, D, and E have the meanings given above, is reacted, optionally after protecting any free hydroxy groups present, with a sulphonyl isocyanate of the general formula III

$$R_6SO_2NCO \qquad III$$

in which $R_6$ represents a phenyl ring that may be substituted by alkyl having 1 to 4 carbon atoms or represents halogen, and then with a tertiary amine or a tertiary amide.

5. Medicaments consisting of one or more compounds according to claim 1 and of customary auxiliaries and carriers.

**Revendications**

1. Dérivés de la prostacycline répondant à la formule générale I:

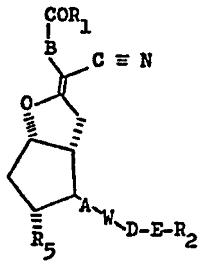

(I)

dans laquelle:

$R_1$ représente un radical $OR_3$ dans lequel $R_3$ peut désigner l'hydrogène ou un alkyle en $C_1$—$C_{10}$, ou encore représente un radical $NHR_4$ dans lequel $R_4$ désigne un radical alcanoyle ou un radical alcane-sulfonyle contenant chacun de 1 à 10 atomes de carbone,

B représente un radical alkylène linéaire contenant de 1 à 5 atomes de carbone,

A représente un groupe —$CH_2$—$CH_2$—, un groupe —CH=CH— cis ou un groupe —CH=CH— trans,

W représente un groupe

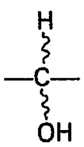 ou un groupe 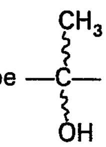

dans lesquels le groupe OH peut être estérifié par un radical d'acide alcanoïque contenant de 1 à 4 atomes de carbone, le groupe hydroxy libre ou estérifié pouvant avoir la configuration $\alpha$ ou $\beta$,

D et E forment ensemble une liaison directe ou

D représente un radical alkylene, linéaire ou ramifié, contenant de 1 à 5 atomes de carbone et

E représente une liaison directe,

$R_2$ représente un radical alkyle saturé, linéaire ou ramifié, contenant de 1 à 6 atomes de carbone, qui peut porter un radical phényle, ou représente un radical phényle, et

$R_5$ représente un groupe hydroxy qui peut être estérifié par un radical d'acide alcanoïque en $C_1$—$C_4$

et également, lorsque $R_3$ désigne l'hydrogène, les sels qu'ils forment avec des bases acceptables du point de vue physiologique.

2. Cyano-5 prostacycline.

3. Cyano-5 méthyl-16 prostacycline.

4. Procédé de préparation de dérivés de la prostacycline de formule générale I, caractérisé en ce qu'on fait réagir, de manière connue, un composé répondant à la formule générale II:

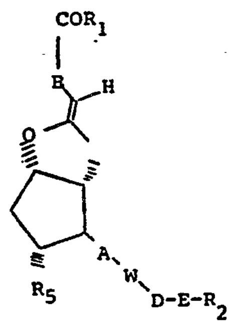

(II)

dans laquelle $R_1$, $R_2$ $R_5$, A, B, W, D et E ont les significations indiquées ci-dessus, éventuellement après avoir protégé d'éventuels groupes hydroxy libres, avec un isocyanate de sulfonyle répondant à la formule générale III:

$$R_6SO_2NCO \qquad (III)$$

dans laquelle $R_6$ représente un noyau phényle éventuellement porteur d'un radical alkyle en $C_1$—$C_4$, ou représente un halogène, puis avec une amine tertiaire ou un amide tertiaire.

5. Médicament constitué d'un ou de plusieurs composés selon la revendication 1 ainsi que d'adjuvants et d'excipients usuels.